# FASCICULE DE BREVET EUROPEEN

(11) **EP 4 007 793 B1**
(45) Date de publication et mention de la délivrance du brevet: **22.02.2023**
(21) Numéro de dépôt: 20754792.8
(22) Date de dépôt: 30.07.2020
(51) Int. Cl.: C08L 23/22, C08L 91/00, C09J 123/22, C09J 191/00, A61F 13/02, A61L 15/58

(54) **COMPOSITION ELASTOMERIQUE**
ELASTOMERZUSAMMENSETZUNG
ELASTOMER COMPOSITION

(30) Priorité: 02.08.2019 FR 1908878
(43) Date de publication de la demande: 08.06.2022
(73) Titulaire: URGO RECHERCHE INNOVATION ET DEVELOPPEMENT, 21300 Chenôve (FR); UNIVERSITE DE PAU ET DES PAYS DE L'ADOUR, 64000 Pau (FR); CENTRE NATIONAL DE LA RECHERCHE SCIENTIFIQUE (CNRS), 75016 Paris (FR)
(72) Inventeur: DERAIL, Christophe, 64170 CESCAU (FR); RONCIN, Armelle, 64990 MOUGUERRE (FR); CALLIES, Xavier, 94130 NOGENT SUR MARNE (FR); GUILLAMAUD, Christelle Marie Aline, 21300 CHENOVE (FR); DANEROL, Anne-Sophie, 21000 DIJON (FR); DAUX, Marie, 21640 Gilly Les Citeaux (FR)
(74) Mandataire: Plasseraud IP
(86) Numéro de dépôt international: PCT/FR2020/051403
(87) Numéro de publication internationale: WO 2021/023929

(56) Documents cités:
- EP-A1- 0 130 061
- EP-A1- 1 676 895
- WO-A2-03/087254
- US-A1- 2011 306 677
- US-A1- 2018 071 150
- US-B1- 6 746 765

## Description

La présente invention est relative à une nouvelle composition à base de copolymères tribloc du type ABA, comportant deux blocs terminaux thermoplastiques A styrène, vitreux à température d'utilisation, et une séquence centrale élastomérique B qui est une oléfine saturée, et d'au moins un polymère linéaire de type polyisobutène de très bas poids moléculaire. La présente composition présente des propriétés d'adhésion et de tenue sur peau intéressantes s'apparentant aux propriétés obtenues au moyen de matrices à base d'élastomères de silicone.

La présente invention est également relative à un pansement comprenant une matrice obtenue à partir d'une telle composition élastomérique.

### Technique antérieure

Les élastomères de silicone sont des composés largement connus. Ils présentent en effet des propriétés physiques et mécaniques exceptionnelles et sont utilisés dans de nombreux domaines (automobile, dispositifs médicaux, puériculture, optique, cosmétique, etc...). Dans le domaine des adhésifs, les élastomères de silicone sont particulièrement intéressants, notamment dans leur application sur la peau, les plaies, les phanères ou les muqueuses. Ce sont des adhésifs « doux », qui ne sont pas agressifs sur la peau au sens où ils sont bien tolérés, tout en présentant une bonne tenue dans le temps de l'adhérence. Ils sont en outre repositionnables, atraumatiques lors de leur retrait et donc bien tolérés par la peau, en particulier les peaux fragiles telles que la peau péri-lésionnelle. Ces adhésifs peuvent être appliqués, retirés puis appliqués à nouveau, et ce sans laisser de résidus ni provoquer de rougeurs. Cependant, les procédés de fabrication des élastomères de silicone sont assez complexes : qu'ils soient obtenus par vulcanisation à chaud ou à froid, leur procédé de fabrication doit respecter des conditions de température et d'humidité très précises. De par la nature des composants entrant dans leur composition et leur procédé d'obtention, les élastomères de silicone sont donc onéreux.

En outre, lorsqu'ils sont destinés à un usage médical, les élastomères de silicone doivent être stérilisés. Or, ces composés ne peuvent être stérilisés que par une méthode bien spécifique à l'oxyde d'éthylène, car leur exposition à tout rayonnement lumineux (constituant une technique classique de stérilisation) entraine une modification de leur structure, résultant en une dégradation de leurs propriétés adhésives.

### Résumé

Ainsi, la présente invention propose de mettre au point une composition spécifique à base d'élastomères non siliconés présentant des propriétés d'adhésion et de tenue sur peau intéressantes, s'approchant des propriétés obtenues au moyen de compositions à base d'élastomères de silicone. De telles compositions, présentant les propriétés d'adhérence recherchées, peuvent en particulier être obtenues sans ajout de résine tackifiante. En effet, pour conférer des propriétés de collant suffisantes aux compositions à base d'élastomères non siliconés, il est généralement nécessaire d'introduire une résine tackifiante. La présence d'une telle résine confère, au toucher, un collant plus sec donc moins doux et plus agressif se traduisant par une moins bonne conformabilité et une adhérence très importante. L'ajout d'une résine peut, de plus, entraîner une instabilité de l'ensemble de la composition.

Plus précisément, il a été découvert, et ceci constitue le fondement de la présente invention, que des compositions mettant en oeuvre au moins un copolymère élastomérique tribloc spécifique, du type styrène - oléfine saturée - styrène, et au moins un polymère de type polyisobutène de très bas poids moléculaire, en une quantité pondérale prédéterminée, permettent de réaliser des matrices élastomériques présentant des propriétés adhésives améliorées, s'apparentant à celles obtenues avec des élastomères de silicone.

L'invention couvre ainsi également une matrice élastomérique obtenue au moyen d'une composition telle que décrite précédemment.

Les matrices élastomériques de l'invention peuvent être obtenues à partir d'un procédé de fabrication classique. Elles peuvent être stérilisées par rayonnement contrairement aux matrices mettant en oeuvre des adhésifs siliconés.

Une fois appliquées, les matrices élastomériques obtenues, pouvant être mises en oeuvre dans divers dispositifs adhésifs tels que par exemple des pansements, se manipulent facilement, sont repositionnables, permettent un retrait sans douleur lorsqu'ils sont appliqués sur la peau, les muqueuses ou les phanères, et présentent une tenue dans le temps satisfaisante.

Ainsi selon un premier aspect, la présente invention a pour objet une composition comprenant :
- 2,5 à 20 % d'un copolymère tribloc styrène - oléfine saturée - styrène
- 45 à 97,5 % en poids d'un polyisobutène de poids moléculaire en nombre compris entre 700 g.mol⁻¹ et 3000 g.mol⁻¹,
les pourcentages étant exprimés en poids, par rapport au poids total de la composition.

Selon un second aspect, la présente invention a pour objet une composition comprenant :
- 4 à 12 % en poids d'un copolymère tribloc styrène - oléfine saturée - styrène,
- 45 à 70 % en poids d'un polyisobutène de poids moléculaire en nombre compris entre 700 g.mol⁻¹ et 3000 g.mol⁻¹,
- 30 à 70 % en poids d'un plastifiant,
les pourcentages étant exprimés en poids, par rapport au poids total de la composition.

En particulier, selon un mode préféré des premier et second aspects de l'invention, la composition comprend en outre 0.05 à 0.4 % en poids d'un agent antioxydant, les pourcentages étant exprimés en poids, par rapport au poids total de la composition. L'agent antioxydant permet d'avoir une composition stable dans le temps.

Selon un troisième aspect, la présente invention a pour objet une matrice élastomérique obtenue à partir d'une telle composition et un pansement comprenant ladite matrice élastomérique.

### Description des modes de réalisation

Copolymère tribloc styrène - oléfine saturée - styrène La composition selon l'invention, comprend au moins un copolymère séquencé tribloc du type ABA (styrène - oléfine saturée - styrène).

Les copolymères séquencés utilisés dans le cadre de l'invention sont des copolymères triblocs du type ABA comportant deux blocs terminaux A styrène (blocs non élastomériques, vitreux à température d'utilisation, en particulier thermoplastiques) et une séquence centrale B oléfine saturée (bloc élastomérique). Ils sont notamment préparés par des techniques de polymérisation anionique ou radicalaire. Les copolymères triblocs peuvent revêtir des structures diverses : linéaire, en étoile (également dénommée radiale), branchée ou encore en peigne.

Par souci de simplicité, dans la présente description, les blocs polymériques constituant les copolymères précités sont désignés par la nature de leurs unités récurrentes. Ainsi, l'expression « bloc » ou « séquence styrène A » désigne une séquence poly(styrène) et l'expression « bloc » ou « séquence oléfine saturée » désigne une séquence poly(oléfine saturée).

Les blocs A sont donc des blocs non élastomériques styréniques (ou polystyréniques).

Les blocs B d'oléfines saturées peuvent par exemple être :
- le polyéthylène hydrogéné suivi d'un bloc polybutylène hydrogéné : le copolymère bloc a alors pour structure: polystyrène-poly(éthylène-butylène)-polystyrène et pour dénomination : SEBS ;
- le polyéthylène hydrogéné suivi d'un bloc polypropylène hydrogéné: le copolymère bloc a alors pour structure : polystyrène-poly(éthylène-propylène)-polystyrène et pour dénomination : SEPS ;
- le polyéthylène hydrogéné suivi d'un bloc polyéthylène hydrogéné puis d'une bloc polypropylène hydrogéné: le copolymère bloc a alors pour structure : polystyrène-poly(éthylène-éthylène-propylène)-polystyrène et pour dénomination : SEEPS ;
- le polyisoprène : le copolymère bloc a alors pour structure : polystyrène-polyisoprène-polystyrène, et pour dénomination : SIS ;
- le polyisoprène suivi d'un bloc polybutadiène : le copolymère bloc a alors pour structure : polystyrène-polyisoprène-polybutadiène-polystyrène, et pour dénomination : SIBS ; ou
- le polybutadiène : le copolymère bloc a alors pour structure : polystyrène-polybutadiène-polystyrène, et pour dénomination : SBS.

Selon un mode préféré de réalisation, les blocs B sont des blocs éthylène-butylène, éthylène-propylène ou éthylène-éthylène-propylène. Les copolymères blocs préférés sont ainsi choisis parmi le polystyrène-poly(éthylène-butylène)-polystyrène (SEBS), le polystyrène-poly(éthylène-propylène)-polystyrène (SEPS) et le polystyrène-poly(éthylène-éthylène-propylène)-polystyrène (SEEPS).

Dans le cadre de la présente invention, on préférera les copolymères triblocs SEBS, SEPS ou SEEPS ayant une teneur en styrène comprise entre 10 et 45 % en poids, de préférence entre 10 et 35 % en poids, par rapport au poids dudit copolymère SEBS, SEPS ou SEEPS.

Les copolymères triblocs à séquence centrale saturée sont bien connus de l'homme de l'art et sont par exemple commercialisés :
- par la société KRATON sous la dénomination KRATON^{®} G, et en particulier les grades KRATON^{®} G1651, KRATON^{®} G1654, KRATON^{®} G1652 ou KRATON^{®} G1650 et par la société KURARAY sous les dénominations SEPTON^{®} et en particulier les grades 8006 ou 8004 pour les copolymères séquencés poly (styrène-éthylène-butylène-styrène) (en abrégé SEBS) ;
- par la société KURARAY sous la dénomination SEPTON^{®} pour les copolymères séquencés poly (styrène-éthylène-propylène-styrène) (en abrégé SEPS) et en particulier les grades 2005, 2006 ou 2063 et pour les polymères séquencés poly (styrène-éthylène-éthylène-propylène-styrène) (en abrégé SEEPS) et en particulier les grades 4033, 4044, 4055, 4077 ou 4099 ;
- par la société Dynasol sous la dénomination Calprène^{®}, en particulier Calprène^{®} H6140 et H6144 pour un copolymère séquencé SEBS.

Selon un mode préféré de réalisation, le copolymère tribloc styrène - oléfine saturée - styrène selon l'invention présente une viscosité mesurée dans une solution à 5 % (masse/masse) dans le toluène à 30 °C, comprise entre 0,01 et 1 Pa.s.

Parmi les copolymères qui présentent une viscosité comprise entre 0,01 et 1 Pa.s mesurée dans une solution à 5 %, on peut citer les copolymères commercialisés par la société KRATON sous les grades KRATON^{®} G1651 et KRATON^{®} G1654 et les copolymères commercialisés par la société KURARAY sous les grades SEPTON^{®} 4055, 4077, 4044 ou 4099.

On peut également citer les copolymères commercialisés par la société Dynasol sous les grades Calprène^{®} H 6140 et Calprène^{®} H6144 ou par la société TSRC sous les grades Taipol^{®} 6151 et Taipol^{®} 6154 et les copolymères commercialisés par la société KURARAY.

Selon un autre mode préféré de réalisation, le copolymère tribloc styrène - oléfine saturée - styrène selon l'invention présente une viscosité mesurée dans une solution à 15 % (masse/masse) dans le toluène à 30 °C, comprise entre 0,01 et 0,5 Pa.s.

Parmi les copolymères qui présentent une viscosité mesurée dans une solution à 15 % comprise entre 0,01 et 0,5 Pa.s, on peut citer les copolymères commercialisés par la société KRATON^{®} sous les grades KRATON^{®} G1650, KRATON^{®} G1657 et KRATON^{®} G1652, KRATON^{®} G1726, et les copolymères commercialisés par la société KURARAY sous les grades SEPTON^{®} 8076 ou 4033. On peut également citer les copolymères commercialisés par la société TSRC sous les grades Taipol^{®} 6150 ou 6152.

Ces viscosités sont mesurées à l'aide d'un viscosimètre Brookfield modèle LVI dans une solution dans le toluène à 5 % ou 15 % masse/masse en fonction du poids moléculaire du copolymère.

De préférence, le copolymère tribloc styrène - oléfine saturée - styrène mis en oeuvre dans le cadre de la présente invention est un polystyrène-poly(éthylène-éthylène-propylène)-polystyrène (SEEPS) tel que de préférence le Septon^{®} 4055 ou un polystyrène-poly(éthylène-butylène)- polystyrène (SEBS), tel que de préférence le KRATON^{®} G1654 ou les Calprène^{®} H6144 et H6140.

De façon générale, la quantité de copolymère tribloc styrène - oléfine saturée - styrène dans la composition est comprise entre 2,5 et 20 % en poids, de préférence entre 4 et 12 % en poids, préférentiellement entre 6 et 10 % en poids, rapportée au poids total de la composition.

Outre le copolymère tribloc styrène-oléfine saturée-styrène, la composition selon l'invention peut également comprendre un copolymère dibloc. De préférence, le copolymère dibloc a pour formule générale AB dans laquelle A et B sont tels que définis précédemment.

Dans le cadre de l'invention, on préfère utiliser un mélange d'un copolymère tribloc et d'un copolymère dibloc ayant les mêmes blocs A et/ou B, en raison notamment du fait que de tels mélanges sont directement disponibles commercialement et garantissent une meilleure miscibilité du mélange.

Selon une variante préférée, la teneur en copolymère dibloc dans le mélange de copolymères diblocs/triblocs styréniques est comprise entre 30 et 95 % en poids, par rapport au poids total du mélange de copolymères diblocs/triblocs styréniques.

En particulier, le mélange de copolymère dibloc/tribloc présente un taux final contrôlé de tribloc/dibloc. Une telle association présente l'avantage d'obtenir une composition présentant une meilleure capacité de dissipation et donc une meilleure adhésivité. La dissipation est la propriété de déformation irréversible de la couche adhésive. En se déformant, ce type d'adhésif consomme de l'énergie qui est dissipée dans la déformation. C'est cette propriété qui permet notamment d'avoir une bonne adhérence, en ce sens qu'il faut une énergie importante pour obtenir cette déformation qui n'est pas emmagasinée par la couche adhésive.

Parmi les mélanges de copolymère dibloc/tribloc connu de l'homme du métier, on peut citer à titre d'exemple le Kraton^{®} G1726 dont le taux de styrène est de 30% ou le Kraton^{®} G1657 commercialisé par la société KRATON dont le taux de styrène est de 13 %.

En particulier, la quantité de copolymère dibloc styrène-oléfine saturée dans la composition est comprise entre 1 et 10 % en poids, de préférence entre 2 et 6 % en poids, préférentiellement entre 2,5 et 5 % en poids, rapportée au poids total de la composition.

Selon un mode particulier de réalisation, les copolymères tribloc/dibloc styréniques sont présents dans la composition en un ratio tribloc/dibloc compris entre 1 et 3, de préférence entre 1,5 et 2.

### Le polyisobutène

Le polyisobutène (PIB) est un homopolymère saturé, peu réactif (faible oxydabilité), issu du monomère isobutylène. Cette polyoléfine a pour motif de répétition -[CH2-C(CH3)2]n-.

Les PIB résultent de la copolymérisation cationique de l'isobutylène (H2C=C(CH3)2, comonomère monoinsaturé) avec l'isoprène (H2C=C(CH3)-CH=CH2, diène conjugué). La réaction s'effectue en solution dans le chlorométhane à -95 °C en présence de chlorure d'aluminium (AlCl3). Les chaînes polymères contiennent environ 1 à 2 % d'unités isopréniques (cis et trans). L'enchaînement en 1,4 du diène laisse une double liaison (insaturation).

Par PIB de très bas poids moléculaire on entend, au sens de la présente invention, des PIB dont le poids moléculaire en nombre se situe entre 700 g.mol⁻¹ et 3000 g.mol⁻¹, de préférence entre 750 g.mol⁻¹ et 1500 g.mol⁻¹, plus préférentiellement entre 800 g.mol⁻¹ et 1400 g.mol⁻¹, encore plus préférentiellement entre 850 g.mol⁻¹ et 1300 g.mol⁻¹, davantage préférentiellement entre 900 g.mol⁻¹ et 1200 g.mol⁻¹.

Le poids moléculaire en nombre du PIB est mesuré par chromatographie d'exclusion stérique selon la méthode suivante :
- Solution de PIB à 2 g.L⁻¹ environ dans du tétrahydrofurane (THF)
- Volume injection : 100 µL
- Débit : 1 mL.min⁻¹
- Détecteur : RI (indice réfraction)
- Température four : 35 °C +/- 5 °C
- Température RI : 40 °C

Les PIB susceptibles d'être mis en oeuvre dans le cadre de la présente invention sont bien connus de l'homme du métier et disponibles commercialement, par exemple sous les dénominations commerciales suivantes :
- TER PIB^{®} 950 commercialisé par TER France, polyisobutène présentant un poids moléculaire en nombre de 950 g.mol⁻¹ ;
- REWOPAL^{®} PIB 1000 commercialisé par EVONIK, polyisobutène présentant un poids moléculaire en nombre de 1000 g.mol⁻¹ ;
- Glissopal^{®} V190 (également connu sous le nom de Safinol^{®} V190 ou Safic-Chem^{®} V190, présentant un poids moléculaire en nombre de 1000 g.mol⁻¹), V500 (présentant un poids moléculaire en nombre de 1300 g.mol⁻¹) ; commercialisés par BASF ;
- Dynapak^{®} 190, Dynapak^{®} 230 commercialisés par Univar, présentant des poids moléculaires en nombre de 1000 et 1050 g.mol⁻¹ respectivement.

Dans le cadre de la présente invention, le PIB est de préférence présent en une quantité de 45 à 97,5 % de PIB en poids, rapportée au poids total de la composition.

Idéalement, la teneur en PIB dans la composition est comprise entre 50 et 60 % en poids par rapport au poids total de la composition. Quand on augmente la quantité de PIB, on augmente le collant (tack), mais on diminue la cohésion.

### Le plastifiant

Selon un mode particulier de réalisation, et notamment lorsque le procédé de fabrication de la matrice élastomérique se fait par voie fondue, le copolymère tribloc styrène - oléfine saturée - styrène et le PIB présents dans la composition selon l'invention, sont associés à au moins un plastifiant.

Les plastifiants susceptibles d'être utilisés sont bien connus et destinés à améliorer les propriétés d'étirement, de souplesse, d'extrudabilité ou de mise en oeuvre du copolymère tribloc styrène - oléfine saturée - styrène. On pourra, à cet effet, utiliser un ou plusieurs plastifiants si nécessaire.

D'une façon générale, en tant que plastifiants, on préférera des composés liquides, compatibles avec la séquence centrale oléfine saturée des copolymères séquencés précités.

Parmi les composés plastifiants susceptibles d'être mis en oeuvre dans les compositions selon l'invention, on citera en particulier les huiles, de préférence minérales.

Alternativement, on peut aussi utiliser des produits de synthèse à base de mélanges liquides d'hydrocarbures saturés comme par exemple les produits commercialisés par la société TOTAL sous la dénomination GEMSEAL^{®} et en particulier le produit GEMSEAL^{®} 60 qui est un mélange isoparaffinique issu d'une coupe pétrolière totalement hydrogénée.

Dans le cadre de la présente invention, on utilisera de préférence des huiles plastifiantes et en particulier des huiles minérales formées de composés paraffiniques ou naphténiques, ou de leurs mélanges, dans des proportions variables.

Des huiles minérales plastifiantes particulièrement préférées sont formées de mélanges de composés paraffiniques et naphténiques, et en particulier de tels mélanges dans lesquels la proportion de composés de nature paraffinique est majoritaire.

Parmi les huiles plastifiantes convenant particulièrement, on peut citer l'huile commercialisée par la société PETRO CANADA sous la référence PURETOL^{®} 9D ou les huiles BLANDOL^{®} et RUDOL^{®} commercialisées par Sonneborn ou encore l'huile Pionier^{®} 2076P ou l'huile Pionier^{®} 7860 commercialisées par Hansen & Rosenthal.

Outre des huiles, le plastifiant peut comprendre de la vaseline. La vaseline mise en oeuvre dans les compositions de l'invention est une vaseline conforme à la Pharmacopée Française disponible commercialement. On peut citer à titre d'exemple la Vaseline Codex A commercialisée par Aiglon.

Dans le cadre de la présente invention, le plastifiant est présent en une quantité de 30 à 70 %, de préférence 30 à 50 %, préférentiellement de 33 à 40 % en poids, rapportée au poids total de la composition.

### Les hydrocolloïdes

Selon un mode de réalisation de l'invention, les compositions selon l'invention peuvent comprendre des particules d'hydrocolloïde.

Ces particules, lorsqu'elles sont utilisées dans une matrice élastomérique destinée à entrer en contact avec la peau ou la plaie, permettent un retrait indolore et le maintien d'un milieu humide au niveau de la plaie afin de favoriser la cicatrisation.

A cet effet, une quantité faible de particules hydrophiles d'un hydrocolloïde est ainsi soit disposée en surface de la matrice élastomérique une fois celle-ci formée soit, de préférence, dispersée de façon homogène au sein de la composition selon l'invention.

Par « hydrocolloïde » ou « particules d'hydrocolloïde », on entend désigner ici tout composé habituellement utilisé par l'homme de l'art pour son aptitude à absorber les liquides aqueux tels que l'eau, le sérum physiologique ou les exsudats d'une plaie.

Comme hydrocolloïdes appropriés, on peut citer par exemple la pectine, les alginates, les gommes végétales naturelles comme en particulier la gomme de Karaya, les dérivés de cellulose tels que les carboxyméthylcelluloses (telle que, par exemple, la BLANOSE^{®} 7H4XFPH commercialisée par Ashland) et leurs sels de métal alcalin tels que le sodium ou le calcium, ainsi que les polymères synthétiques à base de sels de l'acide acrylique, connus sous l'appellation "superabsorbants", comme par exemple les produits commercialisés par la société CIBA Specialty Chemicals sous la dénomination SALCARE^{®} SC91 ainsi que les mélanges de ces composés.

Certains de ces superabsorbants qualifiés de « microcolloïdes » car ils présentent une taille de particules inférieure à 10 micromètres peuvent bien entendu être également utilisés.

Les hydrocolloïdes préférés dans le cadre de la présente invention sont les sels de métal alcalin de la carboxyméthylcellulose, et en particulier la carboxyméthylcellulose de sodium (CMC).

La taille des particules d'hydrocolloïde, par exemple mesurée par granulométrie laser, est généralement comprise entre 50 et 100 µm, avantageusement de l'ordre de 80 µm.

D'une façon générale, la quantité de particules d'hydrocolloïde incorporées dans la composition selon l'invention sera avantageusement inférieure ou égale à 25 % en poids, avantageusement de 2 à 20 % en poids, de préférence de 5 à 18 % en poids, de préférence encore de 10 à 15 % en poids, rapportée au poids total de ladite composition.

Si les particules d'hydrocolloïde sont disposées en surface de la matrice élastomérique une fois celle-ci formée, leur quantité sera de préférence de l'ordre de 1 à 10 % en poids, et plus particulièrement de 2 à 5 % en poids, rapportée au poids total de ladite matrice élastomérique.

### Les antioxydants

La composition selon l'invention peut également comprendre des agents antioxydants.

Par « agents antioxydants », on entend désigner ici les composés couramment employés par l'homme de l'art pour assurer la stabilité des composés entrant dans la formulation des compositions, en particulier vis-à-vis de l'oxygène, la chaleur, l'ozone ou les rayonnements ultra-violets.

Comme exemples d'agents antioxydants appropriés, on peut citer notamment les antioxydants phénoliques comme en particulier les produits commercialisés par la société BASF sous les dénominations IRGANOX^{®} 1010, IRGANOX^{®} 565, IRGANOX^{®} 1076.

D'une façon générale ces agents antioxydants pourront être utilisés seuls ou en association en une quantité de l'ordre de 0,05 à 1 % en poids, de préférence de 0,05 à 0,4 % en poids, rapportée au poids total de la composition.

Dans le cadre de la présente invention, on préférera l'utilisation du produit IRGANOX^{®} 1010 en une quantité comprise entre 0,05 et 0,4 % en poids, rapportée au poids total de la composition.

### Actifs additionnels

La composition selon l'invention peut en outre comprendre une (ou plusieurs) autre(s) substance(s) active(s) permettant d'induire ou d'accélérer la cicatrisation ou pouvant avoir un rôle favorable dans le traitement de la peau ou d'une plaie.

Parmi ces substances actives, on peut citer, en particulier, à titre d'exemples :
- les agents favorisant la cicatrisation tels que le rétinol, la vitamine A, la vitamine E, la N-Acétyl Hydroxyproline, les extraits de Centella Asiatica, la papaïne, le silicone, les huiles essentielles de thym, niaouli, romarin, sauge, l'acide hyaluronique, le sucrose octasulfate de potassium, le sucralfate, l'allantoïne, la metformine ;
- les agents antibactériens tels que les sels ou complexes d'argent (tels les sulfates d'argent, les nitrates d'argent, les sulfamides d'argent ou encore les zéolites à base d'argent), les sels de zinc ou de cuivre, le métronidazole, la néomycine, les pénicillines, l'acide clavulanique, les tétracyclines, la mynocycline, la chlorotétracycline, les aminoglycosides, l'amikacine, la gentamicine, les probiotiques ;
- les antiseptiques tels que la chlorhexidine, le trichlosan, le biguanide, l'hexamidine, le thymol, le lugol, la povidone iodée, le chlorure de benzalkonium et de benzethonium ;
- les anti-douleurs tels que le paracétamol, la codéine, le dextropropoxyphène, le tramadol, la morphine et ses dérivés, les corticoïdes et leurs dérivés ;
- les anesthésiques locaux tels que la lidocaïne, la benzocaïne, la dibucaïne, le chlorhydrate de pramoxine, la bupivacaïne, la mépivacaïne, la prilocaïne, l'étidocaïne ;
- les anti-inflammatoires comme les anti-inflammatoires non stéroïdiens (AINS), l'aspirine ou acide acétylsalicylique, l'ibuprofène, le kétoprofène, le flurbiprofène, le diclofenac, l'acéclophénac, le kétorolac, le méloxicam, le piroxicam, le ténoxicam, le naproxène, l'indométacine, le naproxcinod, le nimésulid, le célécoxib, l'étoricoxib, le parécoxib, le rofécoxib, le valdécoxib, la phénylbutazone, l'acide niflumique, l'acide méfénamique.

Ces agents actifs pourront être utilisés en une quantité de l'ordre de 0,01 à 20 % en poids, de préférence de 1 à 15 % en poids, et de préférence encore de 2 à 10 % en poids, rapportée au poids total de la composition.

La présence d'hydrocolloïdes au sein de la composition favorisera le relargage de ces agents actifs.

Bien entendu, la composition selon l'invention peut aussi comprendre un ou plusieurs autres composés connus pour leur action dans la phase de détersion comme par exemple :
- des enzymes ;
- l'urée.

### Adjuvants

A titre d'adjuvants susceptibles d'être utilisés dans les compositions selon l'invention, on peut citer des composés connus pour favoriser le relargage des agents actifs, comme par exemple les produits Montanox^{®} 80 ou Sepinov^{®} EMT 10 qui sont couramment utilisés dans les produits URGOTUL^{®} qui incorporent des agents actifs.

Ces adjuvants pourront être utilisés en une quantité de l'ordre de 1 à 15 % en poids, rapportée au poids total de la composition.

Selon un mode préféré de réalisation, la composition selon l'invention consiste en:
- 2,5 à 20 % d'un copolymère tribloc styrène - oléfine saturée - styrène
- 45 à 97,5 % en poids d'un polyisobutène de poids moléculaire en nombre compris entre 700 g.mol⁻¹ et 3000 g.mol⁻¹ ,

et optionnellement, de particules d'hydrocolloïde, d'agent antioxydant, et/ou d'une ou plusieurs substance(s) active(s) permettant d'induire ou d'accélérer la cicatrisation ou pouvant avoir un rôle favorable dans le traitement des plaies,
les pourcentages étant exprimés en poids, par rapport au poids total de la composition.

Selon un autre mode préféré de réalisation, la composition selon l'invention consiste en :
- 4 à 12 % en poids d'un copolymère tribloc styrène-oléfine saturée-styrène,
- 45 à 70 % en poids d'un polyisobutène de poids moléculaire en nombre compris entre 700 g.mol⁻¹ et 3000 g.mol⁻¹,
- 30 à 70 % en poids d'un plastifiant,

et optionnellement, de particules d'hydrocolloïde, d'agent antioxydant, et/ou d'une ou plusieurs substance(s) active(s) permettant d'induire ou d'accélérer la cicatrisation ou pouvant avoir un rôle favorable dans le traitement des plaies,
les pourcentages étant exprimés en poids, par rapport au poids total de la composition.

En particulier, les compositions selon l'invention sont exemptes de résines tackifiantes. Par exempte de résine tackifiante, on entend, au sens de la présente demande, que la composition comprend moins de 0,5 % en poids de résine tackifiante, en particulier moins de 0,05 % en poids, et plus préférentiellement moins de 0,005 % en poids.

Parmi les résines tackifiantes, on peut mentionner les résines polyterpènes ou terpènes modifiées, les résines de colophane, les résines hydrocarbonées, les mélanges de résines cycliques, aromatiques et aliphatiques.

Il peut par exemple s'agir de produits commerciaux tels que :
- des résines polycyclopentadiènes hydrogénées commercialisées par la société ARAKAWA Chemical Industries sous la dénomination ARKON^{®}P,
- des résines commercialisées par la société EXXON Chemical sous la dénomination ESCOREZ^{®} et en particulier la série des résines 5000 qui sont hydrogénées,
- une résine de synthèse formée de copolymères en C5/C9 telle que celle commercialisée par la société CRAY VALLEY sous la dénomination WINGTACK^{®}86, ou une résine à base de polyterpène synthétique telle que celle commercialisée par la société CRAY VALLEY sous la dénomination WINGTACK^{®} 10,
- les résines KRISTALEX^{®} et en particulier KRISTALEX^{®}3105SD et F100 commercialisées par la société EASTMAN, ou Sylvares^{®} SA100 (résine à base d'alpha-méthylstyrène) par la société ARIZONA CHEMICAL, ou encore
- les résines Sukorez^{®} de grades SU-90; SU-100; SU- 100S commercialisées par la société Kolon Industries.

Selon un mode préféré de réalisation, les compositions selon l'invention sont exemptes d'élastomères de silicones. Par exempte d'élastomères de silicones, on entend, au sens de la présente demande, que la composition comprend moins de 0,1% en poids d'élastomères de silicones, en particulier moins de 0,01 % en poids, et plus préférentiellement moins de 0,001 % en poids.

### Procédé de préparation des compositions

Les compositions selon l'invention peuvent être préparées par toute technique connue de l'homme du métier.

Selon un mode préféré de réalisation, les compositions selon l'invention peuvent être préparées par voie « solvant » ou par voie « fondue ».

Par voie solvant, on entend, au sens de la présente demande, tout procédé consistant à dissoudre le copolymère tribloc styrène - oléfine saturée - styrène dans un solvant adapté, ledit solvant étant éliminé par évaporation à l'issue du procédé de préparation de la composition.

Par voie fondue, on entend tout procédé consistant à faire fondre le copolymère tribloc styrène - oléfine saturée - styrène pour réaliser le mélange des constituants de la composition. De préférence, le mélange est réalisé dans un mélangeur ou un malaxeur.

Dans le cadre de la présente invention :
- lorsque le procédé de préparation de la composition est effectué par voie « solvant », le PIB est de préférence présent en une quantité de 80 à 97.5 %, de préférence 80 à 95 % en poids, rapportée au poids total de la composition,
- lorsque le procédé de fabrication de la matrice se fait par voie « fondue », le PIB est de préférence présent en une quantité de 45 à 97,5 %, de préférence 50 à 60 %, préférentiellement de 52 à 58 % de PIB en poids, rapportée au poids total de la composition.

Selon un mode préféré de réalisation, lorsque le procédé de préparation de la composition est effectué par voie « solvant », la composition selon l'invention consiste en :
- 2,5 à 20 % d'un copolymère tribloc styrène - oléfine saturée - styrène
- 45 à 97,5 % en poids d'un polyisobutène de poids moléculaire en nombre compris entre 700 g.mol⁻¹ et 3000 g.mol⁻¹ ,

et optionnellement, de particules d'hydrocolloïde, d'agent antioxydant, et/ou d'une ou plusieurs substance(s) active(s) permettant d'induire ou d'accélérer la cicatrisation ou pouvant avoir un rôle favorable dans le traitement des plaies,
les pourcentages étant exprimés en poids, par rapport au poids total de la composition.

Selon un autre mode préféré de réalisation, lorsque le procédé de préparation de la composition est effectué par voie « fondue », la composition selon l'invention consiste en :
- 4 à 12 % en poids d'un copolymère tribloc styrène - oléfine saturée - styrène,
- 45 à 70 % en poids d'un polyisobutène de poids moléculaire en nombre compris entre 700 g.mol⁻¹ et 3000 g.mol⁻¹,
- 30 à 70 % en poids d'un plastifiant,

et optionnellement, de particules d'hydrocolloïde, d'agent antioxydant, et/ou d'une ou plusieurs substance(s) active(s) permettant d'induire ou d'accélérer la cicatrisation ou pouvant avoir un rôle favorable dans le traitement des plaies,
les pourcentages étant exprimés en poids, par rapport au poids total de la composition.

### Matrice élastomérique :

La présente invention a également pour objet une matrice élastomérique obtenue à partir d'une composition telle que décrite précédemment.

En particulier, la matrice élastomérique est obtenue par formation d'une couche mince, c'est-à-dire présentant une épaisseur de 50 µm à 1 mm, de préférence de 150 µm à 400 µm par calandrage, ou par coulage à chaud de ladite composition, selon des méthodes bien connues de l'homme du métier. La matrice élastomérique peut être enduite sur un support de manière à former un dépôt ajouré ou non. Ainsi, lorsque la matrice élastomérique est destinée à être appliquée sur la peau ou sur une plaie, la matrice pourra avantageusement être enduite de manière à former un dépôt ajouré afin d'obtenir la perméabilité souhaitée.

Les matrices élastomériques obtenues dans le cadre de la présente invention présentant des propriétés adhésives améliorées, s'apparentant à celles obtenues avec des élastomères de silicone.

En particulier, les matrices élastomériques selon l'invention présentent un tack à la boucle d'au moins 15 cN.cm⁻¹.

La mesure du tack à la boucle permet d'évaluer la force nécessaire pour arracher un produit auto-adhésif d'un papier bristol à une température de 23 °C ± 2 °C et à une humidité relative de 50 % ± 15 %.

Elle est opérée selon le protocole suivant, mis en oeuvre dans des exemples de la présente demande :

On réalise des matrices élastomériques à partir des compositions à tester à l'aide d'une presse hydraulique selon le protocole suivant :
On a préchauffé à 90 °C les 2 plateaux de la presse hydraulique. Sur le plateau inférieur de la presse on a déposé un film de polyuréthane (PU) et un non tissé en polypropylène, complexés à un liner en papier (la face non tissée en polypropylène étant disposée de façon opposée au plateau inférieur, le papier se trouvant côté plaque). On a déposé sur cette face environ 3,5 g d'une des compositions décrites et on a recouvert cette dernière par un film de polyester siliconé (le côté siliconé étant disposé au contact de la composition). On a placé 2 cales de 0,25 mm entre le polyester et le non tissé aux extrémités du plateau inférieur de la presse et on a soumis l'ensemble à une pression de 200 bars et une température de l'ordre de 90 à 100 °C.
On a laissé refroidir les matrices ainsi réalisées et on a contrôlé leurs épaisseurs avec un micromètre de manière à obtenir une maquette dont l'épaisseur est de l'ordre de 210 à 260 µm sans le polyester siliconé.

On découpe une bande de 20 cm de longueur et 2 cm de largeur. On fait une boucle avec l'adhésif à l'extérieur.

On fixe les extrémités de la boucle dans la mâchoire supérieure du dynamomètre de manière à ce que la boucle mesure 18 cm à partir de la mâchoire. On place l'extrémité de la mâchoire à 140 mm du papier bristol et on fait descendre le dynamomètre de 80 mm à 300 mm.min⁻¹ de manière à ce que l'adhésif de la boucle soit en contact avec le papier bristol (sur toute sa largeur), fixé à l'horizontale dans la mâchoire inférieure.

Après 5 secondes d'attente, on fait remonter le dynamomètre à 300 mm.min⁻¹ en enregistrant la force nécessaire au décollement complet de la boucle.

Selon un autre mode particulier de réalisation, les matrices élastomériques selon l'invention présentent une résistance au cisaillement d'au moins 7 N, de préférence d'au moins 13 N.

La mesure de la résistance au cisaillement a pour objectif de caractériser la cohésion de la matrice, en mesurant sa résistance lorsqu'elle est soumise à un phénomène de cisaillement linéaire. Elle est réalisée sur des matrices élastomériques obtenues à l'aide d'une presse hydraulique selon le protocole suivant, dont les conditions opératoires sont détaillées en exemple :
On a préchauffé les 2 plateaux de la presse hydraulique. Sur le plateau inférieur de la presse on a déposé un film de polyester siliconé (le côté siliconé étant disposé de façon opposée au plateau inférieur). On a déposé sur cette face environ 20 g d'une des compositions décrites et on a recouvert cette dernière par un film plastique anti-adhérent, par exemple un film de polyester siliconé - fluoré (la face siliconée - fluorée étant disposée au contact de la composition). On a placé 2 cales de 1,2 mm entre les 2 films de polyester aux extrémités du plateau inférieur de la presse et on a soumis l'ensemble à une pression de 200 bars et une température de l'ordre de 90 à 100 °C.
Pour la mesure de la résistance au cisaillement, on a laissé refroidir les plaques ainsi réalisées, et on a contrôlé leurs épaisseurs avec un micromètre de manière à obtenir une maquette dont l'épaisseur de la composition est comprise entre 0,98 mm et 1,08 mm.

Les matrices élastomériques selon l'invention présentent un tack à la boucle d'au moins 15 cN.cm⁻¹ et une résistance au cisaillement d'au moins 7 N, de préférence d'au moins 13 N.

Bien évidemment les modes de réalisation particuliers qui viennent d'être décrits peuvent être mis en oeuvre séparément ou selon l'une quelconque de leurs combinaisons.

Les compositions selon l'invention permettent en particulier de réaliser des matrices élastomériques présentant une adhésivité acceptable et un retrait sans douleur lorsqu'elles sont appliquées sur la peau, la plaie, la muqueuse ou les phanères.

La présente invention est illustrée dans les exemples non limitatifs présentés ci-après.

### Exemples

### Préparation des compositions

Les compositions des exemples 1 à 13 ont été élaborées à l'aide des constituants suivants dans les proportions, exprimées en pourcentage en poids, mentionnées dans le tableau 1 ci-dessous.

### Elastomère :

Copolymère séquencé de poly (styrène-éthylène-butylène-styrène) (en abrégé SEBS) :
- KRATON^{®} G1654 commercialisé par Kraton Polymer
- KRATON^{®} G1651 commercialisé par Kraton Polymer.
- CALPRENE^{®} H6140 commercialisé par Dynasol.

Mélange de copolymère séquencé tribloc de poly (styrène-éthylène-butylène-styrène) (en abrégé SEBS) et de copolymère séquencé dibloc de poly(styrène-éthylène-butylène) (en abrégé SEB) :
- KRATON^{®} G1726 commercialisé par Kraton Polymer.

Copolymère séquencé de poly (styrène-éthylène- éthylène/propylène-styrène) (en abrégé SEEPS) :
- SEPTON^{®} 4055 commercialisé par KURARAY

Plastifiant :
- huiles minérales Pionier^{®} 2076P, Pionier^{®} 1155 ou Pionier^{®} 7860 commercialisées par Hansen & Rosenthal
- huile minérale Rudol^{®} commercialisée par Sonneborn.

Antioxydant : IRGANOX^{®} 1010 commercialisé par la société BASF.

PIB de très bas poids moléculaire :
- Glissopal^{®} V190, Safinol^{®} V190 ou Safi-Chem^{®} V190 (nouveau nom commercial du Safinol V190), commercialisés par SAFIC, présentant un poids moléculaire Mn de 1000 g.mol⁻¹,
- Rewopal^{®} PIB1000 commercialisés par EVONIK, présentant un poids moléculaire Mn de 1000 g.mol⁻¹,

### PIB de bas poids moléculaire :

- Oppanol^{®} B10 SFN commercialisé par BASF, présentant un poids moléculaire en nombre Mn de 18000 g.mol⁻¹.

### Fabrication de la composition par voie fondue :

Dans un mélangeur vertical, on a introduit successivement à une température de consigne de 80 °C le plastifiant et le PIB et on a agité jusqu'à l'obtention d'un mélange homogène.

On a ensuite introduit sous agitation le(s) copolymère(s) et l'antioxydant, puis on a porté la température de consigne à 150 °C et agité jusqu'à l'obtention d'un mélange homogène.

On a ensuite laissé refroidir, puis on a vidangé le mélangeur.

Par la suite, on a réalisé des matrices élastomériques à partir des compositions à tester (compositions 1 à 13), en appliquant une forte pression à l'aide d'une presse hydraulique selon le protocole suivant :
On a préchauffé à 90 °C les 2 plateaux de la presse hydraulique. Sur le plateau inférieur de la presse on a déposé un film de polyuréthane (PU) et un non tissé en polypropylène (la face non tissée en polypropylène étant disposée de façon opposée au plateau inférieur). On a déposé sur cette face environ 3,5 g d'une des compositions décrites et on a recouvert cette dernière par un film de polyester siliconé (le côté siliconé étant disposé au contact de la composition). On a placé 2 cales de 0,25 mm entre les 2 films de polyester aux extrémités du plateau inférieur de la presse et on a soumis l'ensemble à une pression de 200 bars et une température de l'ordre de 90 à 100 °C.
On a laissé refroidir les matrices ainsi réalisées, retiré le liner papier situé sur le film PU, sur la face opposée au non-tissé, et on a contrôlé leurs épaisseurs avec un micromètre de manière à obtenir une maquette dont l'épaisseur est de l'ordre de 210 à 260 µm sans le polyester siliconé.

### Fabrication des compositions 14 et 15 par voie solvant :

Dans un bécher, on a introduit une quantité de toluène équivalente à 5 fois la quantité de matière sèche pour une dissolution complète des polymères et copolymères et obtention d'une solution finale diluée limpide. Le polymère est introduit en premier et après dissolution de celui-ci, on introduit le copolymère. Les deux sont découpés en petites morceaux avant introduction dans le toluène. Les quantités de copolymère et de PIB figurant au [tableau 1] ci-après, et la géométrie du moule permettent de définir en particulier l'épaisseur du film nécessaire pour les analyses ultérieures. Afin d'assurer la mise en solution totale des phases solides, le mélange est agité avec un agitateur magnétique et un barreau aimanté durant toute l'opération à une vitesse moyenne assurant un mélange actif sans projection. La phase de dissolution est opérée à température ambiante, durant 12 heures maximum et sous hotte.

Après cette phase de dissolution, l'agitation est stoppée et le contenu du bécher est versé dans un moule muni d'un revêtement anti-adhérent type téflon. Un film d'aluminium perforé est alors disposé sur le moule en téflon. Ce moule est placé sous une hotte à flux laminaire, à température ambiante, pour évaporation du solvant. Après 24 heures dans ces conditions, le moule est placé 1 heure à 90°C dans une enceinte ventilée. Le moule est ensuite placé à température ambiante durant 2 heures. Le film de polymère est alors récupéré. Il permet la confection d'échantillons pour différentes les différentes caractérisations.

### Mesure du pouvoir adhésif à 180° :

Cette méthode a pour objectif de mesurer la force requise pour décoller d'un support par pelage à 180°, un matériau enduit d'un adhésif sensible à la pression à une vitesse v donnée après un temps de contact t donné à une température de 23 °C ± 2 °C et à une humidité relative de 50 % ± 15 %.

Les matrices obtenues au moyen des compositions selon les exemples 1 à 12 sont appliquées sur un support comme décrit ci-dessus puis découpées à l'aide d'un emporte-pièce afin d'obtenir des éprouvette de largeur l = 20 mm.

Une bande de papier bristol Exacompta 13356E de longueur et largeur supérieures à l'éprouvette sont découpées.

L'éprouvette est appliquée, avec une légère pression du doigt sur le bristol parallèlement à sa plus grande dimension, sans étirer la bande et en évitant d'inclure des bulles d'air. On effectue alors deux allers retours à l'aide d'un rouleau applicateur (de diamètre 85 mm recouvert d'un revêtement caoutchouc de 6 mm d'épaisseur et de masse 500 g) à une vitesse v1 = 10 mm.s⁻¹ et un poids P = 2 kg.cm⁻¹, sans pression additionnelle, pour obtenir un contact intime entre la matrice adhésive et la surface du papier.

Immédiatement après, on effectue la mesure d'adhésivité en positionnant la bande de bristol contre une plaque, le tout maintenu dans la mâchoire inférieure d'un dynamomètre électronique et on fait la mesure de pelage à 180° à la vitesse v2 = 300 mm.min⁻¹.

Le pouvoir adhésif PA = F/I avec :
I = largeur de l'éprouvette en cm
F = force mesurée en N.

Dans le cadre de l'invention, un PA > 10 cN.cm⁻¹ est privilégié.

### Mesure du tack à la boucle

Cette méthode permet d'évaluer la force nécessaire pour arracher un produit auto-adhésif d'un papier bristol à une température de 23 °C ± 2 °C et à une humidité relative de 50 % ± 15 %.

On découpe une bande de 20 cm de longueur par 2 cm. On fait une boucle avec l'adhésif à l'extérieur.

On fixe les extrémités de la boucle dans la mâchoire supérieure du dynamomètre de manière à ce que la boucle mesure 18 cm à partir de la mâchoire. On place l'extrémité de la mâchoire à 140 mm du papier bristol et on fait descendre le dynamomètre de 80 mm à 300 mm.min⁻¹ de manière à ce que l'adhésif de la boucle soit en contact avec le papier bristol (sur toute sa largeur), fixé à l'horizontale dans la mâchoire inférieure.

Après 5 secondes d'attente, on fait remonter le dynamomètre à 300 mm.min⁻¹ en enregistrant la force nécessaire au décollement complet de la boucle.

Les matrices élastomériques selon l'invention présentent un tack à la boucle d'au moins 15 cN.cm⁻¹.

### Mesure de la résistance au cisaillement

Par la suite, on a réalisé des matrices polymériques à partir des compositions à tester, en appliquant une forte pression à l'aide d'une presse hydraulique selon le protocole suivant :
On a préchauffé les 2 plateaux de la presse hydraulique. Sur le plateau inférieur de la presse on a déposé un film de polyester siliconé (le côté siliconé étant disposé de façon opposée au plateau inférieur). On a déposé sur cette face environ 20 g d'une des compositions décrites et on a recouvert cette dernière par un film plastique anti-adhérent, par exemple un film de polyester siliconé - fluoré (la face siliconée - fluorée étant disposée au contact de la composition). On a placé 2 cales de 1,2 mm entre les 2 films de polyester aux extrémités du plateau inférieur de la presse et on a soumis l'ensemble à une pression de 200 bars et une température de l'ordre de 90 à 100 °C.

On a laissé refroidir les plaques ainsi réalisées, et on a contrôlé leurs épaisseurs avec un micromètre de manière à obtenir une maquette dont l'épaisseur de la composition est de l'ordre du mm compris entre 0,98 mm et 1,08 mm.

Cette méthode a pour objectif de caractériser la résistance de certains matériaux lorsqu'ils sont soumis à un phénomène de cisaillement linéaire.

### APPAREILLAGE, MATERIELS, REACTIFS

- Dynamomètre
- Plaquettes : deux plaquettes en acier inoxydable rectangulaires par éprouvette à tester (dimension possible : 25 x 100 x 2 mm)
- Matériau de maintien : Complexe adhésif double face + Teslin SP600 commercialisé par la société PPG Teslin.
- Cales de compensation : deux cales d'épaisseur connue et identique par échantillon à tester (ou association de cales d'épaisseur permettant d'obtenir deux fois la même épaisseur). Chaque cale ou association doit être de l'épaisseur de l'échantillon à tester et du matériau de maintien de l'échantillon le cas échéant.

### ECHANTILLONNAGE ET/OU CONDITIONNEMENT

### Nombre d'échantillons ≥ 3

Conditionnement des échantillons pendant au moins 24h à 23 °C ± 2 °C et 50 % ± 15 % d'Humidité Relative.

### MODE OPERATOIRE

### Préparation des éprouvettes :

Coller le matériau de maintien sur une extrémité de la première plaquette métallique de sorte qu'il recouvre la plaquette métallique sur une longueur L = 20 mm.

Puis appuyer afin de bien faire adhérer le matériau de maintien et découper le surplus au ras de la plaquette métallique.

Répéter l'opération sur la deuxième plaquette métallique (longueur du matériau de maintien identique à la première plaquette).

Découper une bande de l'échantillon à tester (d'épaisseur 1mm) de largeur l = 25 mm (largeur identique à celle des plaquettes métalliques).

Coller l'échantillon à tester sur le matériau de maintien d'une plaquette métallique, puis découper le surplus de la bande d'échantillon au ras de la plaquette métallique de sorte que l'échantillon à tester soit bien de longueur L = 20 mm, et coller sur l'échantillon la zone avec le matériau de maintien de la seconde plaquette métallique.

### Mesure :

Marquer les plaquettes métalliques à la limite métal/ échantillon Appliquer un poids sur la zone à tester (zone de 20 x 25 mm) :
- 1 kg pendant 15 secondes

Fixer délicatement le dispositif avec les cales de compensation dans les mâchoires du dynamomètre (faire attention à l'épaisseur des cales de compensation, tout le dispositif doit être plan afin que la contrainte de cisaillement s'exerce bien dans le plan vertical).

Tester l'éprouvette :
- 45 secondes après avoir retiré le poids

Procéder à l'essai de cisaillement jusqu'à la rupture de l'éprouvette à la vitesse :
- v = 10 mm.min⁻¹ ± 0,5 mm.min⁻¹

Enregistrer la courbe force/déplacement

Vérifier que l'échantillon n'a pas glissé sur les plaquettes à l'aide des marques réalisées précédemment

Vérifier qu'il s'agit bien d'une rupture de cohésion.

### EXPRESSION DES RESULTATS :

Le résultat s'exprime sous la forme de la résistance nécessaire à la rupture de l'éprouvette en N (à 10⁻¹ près).

Les matrices élastomériques selon l'invention présentent une résistance au cisaillement d'au moins 7 N, de préférence d'au moins 13 N.

**[Tableau 1]**

| | Ex.1 | Ex.2 | Ex. 3 | Ex. 4 | Ex. 5 | Ex. 6 | Ex. 7 | Ex. 8 | Ex. 9 | Ex. 10 | Ex. 11 | Ex. 12 | Ex. 13 | Ex. 14 | Ex. 15 |
|---|---|---|---|---|---|---|---|---|---|---|---|---|---|---|---|
| Kraton^{®} G1654 | | | 6 | 6 | 6 | | 6 | 6 | | 6.4 | | | | | |
| Kraton^{®} G1651 | 6 | 6 | | | | | | | | | | | 6 | | |
| Kraton^{®} G1657 | | | | | | | | | | | | | | | 9 |
| Kraton^{®} G1726 | | | | | | | | | | | 5 | 5 | | 19 | |
| Septon^{®} 4055 | | | | | | | | | 6 | | | | | | |
| Calprène^{®} H6140 | | | | | | 7 | | | | | 5 | 5 | | | |
| Safinol^{®} V190 | | | | | 55 | 55 | 55 | 55 | 47 | 55 | | 55 | | 81 | 91 |
| Rewopal^{®} PIB 1000 | | | | | | | | | | | 55 | | | | |
| Oppanol^{®} B10 SFN | | | | | | | | | | | | | 24 | | |
| Pionier^{®} 2076 | 93.8 | | 93.8 | | | | | 38.8 | 46.8 | | | | 69.8 | | |
| Pionier^{®} 1155 | | | | | | | | | | | | 34.8 | | | |
| Pionier^{®} 7860 | | 93.8 | | 93.8 | 38.8 | 37.8 | | | | 38.4 | | | | | |
| Rudol^{®} | | | | | | | 38.8 | | | | 34.8 | | | | |
| Irganox^{®} 1010 | 0.2 | 0.2 | 0.2 | 0.2 | 0.2 | 0.2 | 0.2 | 0.2 | 0.2 | 0.2 | 0.2 | 0.2 | 0.2 | | |
| Sensation sur peau | Non collant | Non collant | Non collant | Non collant | Collant, repositi onnable | Collant, repositi onnable | Collant, repositi onnable | Collant, repositi onnable | Collant, repositi onnable | Collant, repositi onnable | Collant, repositi onnable | Collant, repositi | Mélang e liquide à froid | Collant repositi onnable | Collant repositi onnable |
| Pouvoir adhésif à 180° (cN.cm⁻¹) | Non mesuré | Non mesuré | Non mesuré | Non mesuré | Rupture de Cohési on Superfi cielle | 37.4 | 31.1 | 27.5 | 15.4 | 34.4 | 50.9 | 68.8 | Non mesuré car non homog ène et non cohésif | 100.1 | 120.1 |
| Tack à la boucle (cN.cm⁻¹) | 6.9 | Non mesuré | Non mesuré | Non mesuré | 45.7 | 47.6 | 44.4 | 31.9 | 15.8 | 41.1 | 68.8 | 76.3 | Non mesuré car non homog ène et non cohésif | 96.4 | 171.5 |
| Résistance au cisaillemen t (N) | 30.50 | Non mesuré | Non mesuré | Non mesuré | 13.53 | 11.00 | 14.24 | 10.72 | 10.27 | 13.06 | 14.26 | 14.24 | Non mesuré car non homog ène et non cohésif | 14.23 | 11.31 |

Les matrices élastomériques selon l'invention présentent un tack à la boucle d'au moins 15 cN.cm⁻¹, leur résistance au cisaillement est d'au moins 7 N, de préférence d'au moins 13 N.

Les matrices obtenues au moyen des compositions des exemples 1 à 4 (comparatifs) ne comprennent pas de PIB de très bas poids moléculaires. Ces matrices sont déceptives car elles ne sont pas collantes sur peau.

Les matrices obtenues au moyen des compositions des exemples 5 à 12 sont des matrices selon l'invention. Ces matrices ont été testées en tenue sur peau.

La matrice obtenue, issue de l'exemple 13 formulée avec un PIB de bas poids moléculaires (18000 g.mol⁻¹) et non de très bas poids moléculaire (inférieur à 3000 g.mol⁻¹) comme utilisé dans la présente invention est une matrice liquide à froid. Ainsi, on démontre que les matrices connues de l'art antérieur utilisant des PIB de bas poids moléculaire diffèrent des matrices de la présente invention.

Idéalement, la teneur en PIB dans la composition est de 55 % en poids par rapport au poids total de la composition. Quand on augmente la quantité de PIB, on augmente le tack, mais on perd en cohésion.

### Exemple 16 : Test sur peau

Les exemples 5 à 12 ont été testés sur peau. Les matrices ont été enduites comme pour la mesure du pouvoir adhésif.

Des éprouvettes de 4 cm * 1 cm ont ensuite été découpées. Ces éprouvettes ont été appliquées sur l'avant-bras en apposant la main à une pression d'environ 5 à 15 mmHg.

Le bras est ensuite retourné à l'horizontale (l'éprouvette se trouvant ainsi dirigée côté sol) pour vérifier la tenue de l'éprouvette.

Aucune des éprouvettes enduites des matrices des exemples 5 à 12 n'est tombée.

On repositionne ensuite deux fois l'éprouvette (l'éprouvette est retirée, puis appliquée en apposant la main à une pression d'environ 5 à 15 mmHg les deux fois).

On retourne le bras à l'horizontale. Aucune des éprouvettes enduites des matrices des exemples 5 à 12 n'est tombée.

## Revendications

1. Composition comprenant :
- 2,5 à 20 % d'un copolymère tribloc styrène - oléfine saturée - styrène
- 45 à 97,5 % en poids d'un polyisobutène de poids moléculaire en nombre compris entre 700 g.mol⁻¹ et 3000 g.mol⁻¹ ,
les pourcentages étant exprimés en poids, par rapport au poids total de la composition.

2. Composition selon la revendication 1, **caractérisée en ce que** la quantité en copolymère tribloc styrène - oléfine saturée - styrène est comprise entre 4 et 12 % en poids, préférentiellement entre 6 et 10 % en poids, rapportée au poids total de la composition.

3. Composition selon l'une quelconque des revendications 1 ou 2, **caractérisée en ce que** le copolymère tribloc du type styrène-oléfine saturée-styrène est un SEBS ou un SEEPS.

4. Composition selon l'une quelconque des revendications 1 à 3, **caractérisée en ce que** le PIB est présent en une quantité de 50 à 60 %, préférentiellement de 52 à 58 % de PIB en poids, rapportée au poids total de la composition.

5. Composition selon l'une quelconque des revendications 1 à 4, **caractérisée en ce que** le poids moléculaire en nombre du PIB se situe entre 750 g.mol⁻¹ et 1500 g.mol⁻¹, de préférence entre 800 g.mol⁻¹ et 1400 g.mol⁻¹, plus préférentiellement entre 850 g.mol⁻¹ et 1300 g.mol⁻¹, encore plus préférentiellement entre 900 g.mol⁻¹ et 1200 g.mol⁻¹.

6. Composition comprenant :
- 4 à 12 % en poids d'un copolymère tribloc styrène - oléfine saturée - styrène,
- 45 à 70 % en poids d'un polyisobutène de poids moléculaire en nombre compris entre 700 g.mol⁻¹ et 3000 g.mol⁻¹,
- 30 à 70 % en poids d'un plastifiant,
les pourcentages étant exprimés en poids, par rapport au poids total de la composition.

7. Composition selon la revendication 6, **caractérisée en ce que** le plastifiant est présent en une quantité de 30 à 50 %, préférentiellement de 33 à 40 % en poids, rapportée au poids total de la composition.

8. Composition selon l'une quelconque des revendications 6 ou 7, **caractérisée en ce que** le plastifiant est une huile, de préférence une huile minérale.

9. Composition selon l'une quelconque des revendications 1 à 7, **caractérisée en ce qu'**elle comprend des particules d'hydrocolloïde en une quantité inférieure ou égale à 25 % en poids, avantageusement de 2 à 20 % en poids, de préférence de 5 à 18 % en poids, de préférence encore de 10 à 15 % en poids, rapportée au poids total de la composition.

10. Composition selon l'une des revendications précédentes, **caractérisée en ce qu'**elle comprend une ou plusieurs substance(s) active(s) permettant d'induire ou d'accélérer la cicatrisation ou pouvant avoir un rôle favorable dans le traitement des plaies, en une quantité comprise entre 0,01 et 20 % en poids, de préférence entre 1 et 15 % en poids, rapportée au poids total de la composition.

11. Matrice élastomérique, **caractérisée en ce qu'**elle est obtenue à partir d'une composition selon l'une des revendications 1 à 10, de préférence par formation d'une couche présentant une épaisseur de 50 µm à 1 mm, de préférence de 150 µm à 400 µm par calandrage, ou par coulage à chaud de ladite composition.

12. Pansement **caractérisé en ce qu'**il comprend une matrice élastomérique selon la revendication 11.

## Patentansprüche

1. Zusammensetzung, umfassend:
- 2,5 bis 20 % eines Triblock-Copolymers Styrol-gesättigtes Olefin-Styrol,
- 45 bis 97,5 Gew.-% eines Polyisobutens mit einer Molekulargewichtszahl von 700 g.mol⁻¹ bis 3000 g.mol⁻¹,
wobei die Prozentangaben in Gewichtsprozent, bezogen auf das Gesamtgewicht der Zusammensetzung, angegeben sind.

2. Zusammensetzung nach Anspruch 1, **dadurch gekennzeichnet, dass** die Menge des Triblock-Copolymers Styrol-gesättigtes Olefin-Styrol zwischen 4 und 12 Gew.-%, bevorzugt zwischen 6 und 10 Gew.-%, bezogen auf das Gesamtgewicht der Zusammensetzung, beträgt.

3. Zusammensetzung nach einem der Ansprüche 1 oder 2, **dadurch gekennzeichnet, dass** das Triblock-Copolymer vom Typ Styrol-gesättigtes Olefin-Styrol ein SEBS oder ein SEEPS ist.

4. Zusammensetzung nach einem der Ansprüche 1 bis 3, **dadurch gekennzeichnet, dass** das PIB in einer Menge von 50 bis 60 %, bevorzugt 52 bis 58 Gewichtsprozent PIB, bezogen auf das Gesamtgewicht der Zusammensetzung, vorliegt.

5. Zusammensetzung nach einem der Ansprüche 1 bis 4, **dadurch gekennzeichnet, dass** die Molekulargewichtszahl des PIB zwischen 750 g.mol⁻¹ und 1500 g.mol⁻¹, bevorzugt zwischen 800 g.mol⁻¹ und 1400 g.mol⁻¹, besonders bevorzugt zwischen 850 g.mol⁻¹ und 1300 g.mol⁻¹, noch stärker bevorzugt zwischen 900 g.mol⁻¹ und 1200 g.mol⁻¹ liegt.

6. Zusammensetzung, umfassend:
- 4 bis 12 Gew.-% eines Triblock-Copolymers Styrol-gesättigtes Olefin-Styrol,
- 45 bis 70 Gew.-% eines Polyisobutens mit einer Molekulargewichtszahl zwischen 700 g.mol⁻¹ und 3000 g.mol⁻¹,
- 30 bis 70 Gew.-% eines Weichmachers,
wobei die Prozentangaben in Gewichtsprozent, bezogen auf das Gesamtgewicht der Zusammensetzung, angegeben sind.

7. Zusammensetzung nach Anspruch 6, **dadurch gekennzeichnet, dass** der Weichmacher in einer Menge von 30 bis 50 Gew.-%, bevorzugt 33 bis 40 Gew.-%, bezogen auf das Gesamtgewicht der Zusammensetzung, vorliegt.

8. Zusammensetzung nach einem der Ansprüche 6 oder 7, **dadurch gekennzeichnet, dass** der Weichmacher ein Öl, bevorzugt ein Mineralöl, ist.

9. Zusammensetzung nach einem der Ansprüche 1 bis 7, **dadurch gekennzeichnet, dass** sie Hydrokolloidteilchen in einer Menge kleiner gleich 25 Gew.-%, vorteilhafterweise 2 bis 20 Gew.-%, bevorzugt 5 bis 18 Gew.-%, noch stärker bevorzugt 10 bis 15 Gew.-%, bezogen auf das Gesamtgewicht der Zusammensetzung, enthält.

10. Zusammensetzung nach einem der vorhergehenden Ansprüche, **dadurch gekennzeichnet, dass** sie in einer Menge von 0,01 bis 20 Gew.-%, bevorzugt 1 bis 15 Gew.-%, bezogen auf das Gesamtgewicht der Zusammensetzung einen oder mehrere Wirkstoffe enthält, die es ermöglichen, die Wundheilung zu induzieren oder zu beschleunigen oder eine günstige Rolle bei der Behandlung von Wunden spielen können.

11. Elastomermatrix, **dadurch gekennzeichnet, dass** sie aus einer Zusammensetzung nach einem der Ansprüche 1 bis 10 erhalten wird, bevorzugt durch Bildung einer Schicht mit einer Dicke von 50 µm bis 1 mm, bevorzugt von 150 µm bis 400 µm durch Kalandrieren oder durch Heißgießen der Zusammensetzung.

12. Wundverband, **dadurch gekennzeichnet, dass** er eine Elastomermatrix nach Anspruch 11 umfasst.

## Claims

1. Composition comprising:
- 2.5 to 20% of a styrene - saturated olefin - styrene triblock copolymer
- 45 to 97,5% by weight of a polyisobutene with a number molecular weight of between 700 g.mol⁻¹ and 3000 g.mol⁻¹,
the percentages being expressed by weight, relative to the total weight of the composition.

2. Composition according to claim 1, **characterized in that** the amount of styrene - saturated olefin - styrene triblock copolymer is between 4 and 12% by weight, preferably between 6 and 10% by weight, relative to the total weight of the composition.

3. Composition according to any one of claims 1 or 2, **characterized in that** the triblock copolymer of the styrene - saturated olefin - styrene type is a SEBS or a SEEPS.

4. Composition according to any one of claims 1 to 3, **characterized in that** the PIB is contained in an amount of 50 to 60%, preferably 52 to 58% PIB by weight, relative to the total weight of the composition.

5. Composition according to any one of claims 1 to 4, **characterized in that** the number molecular weight of the PIB is between 750 g.mol⁻¹ and 1500 g.mol⁻¹, preferably between 800 g.mol⁻¹ and 1400 g.mol⁻¹, more preferably between 850 g.mol⁻¹ and 1300 g.mol⁻¹, even more preferably between 900 g.mol⁻¹ and 1200 g.mol-1

6. Composition comprising:
- 4 to 12% by weight of a styrene - saturated olefin - styrene triblock copolymer,
- 45 to 70% by weight of a polyisobutene with a number molecular weight of between 700 g.mol-1 and 3000 g.mol⁻¹,
- 30 to 70% by weight of a plasticizer,
the percentages being expressed by weight, relative to the total weight of the composition.

7. Composition according to claim 6, **characterized in that** the plasticizer is contained in an amount of 30 to 50%, preferably 33 to 40% by weight, relative to the total weight of the composition.

8. Composition according to any one of claims 6 or 7, **characterized in that** the plasticizer is an oil, preferably a mineral oil.

9. Composition according to any one of claims 1 to 7, **characterized in that** it comprises hydrocolloid particles in an amount less than or equal to 25% by weight, advantageously of 2 to 20% by weight, preferably of 5 to 18% by weight, more preferably 10 to 15% by weight, relative to the total weight of the composition.

10. Composition according to one of the preceding claims, **characterized in that** it comprises one or several active substance(s) making it possible to induce or accelerate cicatrization or which can have a favorable role in the wound treatment, in an amount of between 0.01 and 20% by weight, preferably between 1 and 15% by weight, relative to the total weight of the composition.

11. Elastomeric matrix, **characterized in that** it is obtained from a composition according to any one of claims 1 to 10, preferably by forming a layer having a thickness of 50 µm to 1 mm, preferably of 150 µm to 400 µm by calendering, or by hot casting of said composition.

12. Dressing **characterized in that** it comprises an elastomeric matrix according to claim 11.
